(19) Europäisches Patentamt
European Patent Office
Office européen des brevets

(11) **EP 3 433 607 B1**

(12) **EUROPEAN PATENT SPECIFICATION**

(45) Date of publication and mention of the grant of the patent:
**27.04.2022 Bulletin 2022/17**

(21) Application number: **17716054.6**

(22) Date of filing: **23.03.2017**

(51) International Patent Classification (IPC):
**G01F 1/66** *(2022.01)*     **G01N 33/28** *(2006.01)*
**G01F 1/74** *(2006.01)*     **G01F 1/704** *(2006.01)*
**G01F 25/00** *(2022.01)*

(52) Cooperative Patent Classification (CPC):
**G01F 1/74; G01F 1/661; G01F 1/704; G01N 33/2888;** G01F 25/10

(86) International application number:
**PCT/IB2017/051678**

(87) International publication number:
**WO 2017/163207 (28.09.2017 Gazette 2017/39)**

(54) **PROCESS AND SYSTEM FOR CONTROLLING SPRAYED LUBRICATION WITH INSTANTANEOUS MEASUREMENT OF THE LUBRICANT FLOW**

VERFAHREN UND SYSTEM ZUR STEUERUNG VON GESPRÜHTER SCHMIERUNG MIT UNMITTELBARER MESSUNG DES SCHMIERMITTELFLUSSES

PROCÉDÉ ET SYSTÈME DE COMMANDE DE LUBRIFICATION PULVÉRISÉE AVEC MESURE INSTANTANÉE DU FLUX DE LUBRIFIANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.03.2016 IT UA20162029**

(43) Date of publication of application:
**30.01.2019 Bulletin 2019/05**

(73) Proprietors:
• **MWM Schmieranlagen S.r.L.**
**20068 Peschiera Borromeo (Milano) (IT)**
• **Politecnico di Milano**
**20133 Milano (IT)**

(72) Inventors:
• **NORGIA, Michele**
**20131 Milano (IT)**
• **MAZZONI, Maurizio**
**20135 Milano (IT)**

(74) Representative: **Ripamonti, Enrico et al**
**Giambrocono & C. S.p.A.**
**Via Rosolino Pilo, 19/B**
**20129 Milano (IT)**

(56) References cited:
EP-A1- 2 837 327          WO-A1-2004/079254
DE-A1-102006 030 651     US-A1- 2012 004 865

• **CHRISTIAN ZAKIAN ET AL: "Particle sizing and flow measurement using self-mixing interferometry with a laser diode; Particle sizing and flow measurement using self-mixing interferometry", JOURNAL OF OPTICS. A, PURE AND APPLIED OPTICS, INSTITUTE OF PHYSICS PUBLISHING, BRISTOL, GB, vol. 7, no. 6, 1 June 2005 (2005-06-01), pages S445-S452, XP020093128, ISSN: 1464-4258, DOI: 10.1088/1464-4258/7/6/029**

**EP 3 433 607 B1**

**Description**

This invention relates to a method for measuring spray lubrication according to claim 1.

The invention also relates to a system for implementing the abovementioned method in accordance with claim 10.

Systems and corresponding devices for lubricating mechanical components/mechanisms (bearings, gear mechanisms) or tools for mechanical machining with the removal of swarf (milling, drilling, thread cutting or the like) by means of an oil mist lubricating process (using an aerosol or spray lubrication) have been known for some time. This spray lubrication process for example comprises the passage of a lubricating fluid containing an oil mist into a mechanical member (for example a chuck) and the exit of such lubricant from at least one suitable point provided in such member towards a component thereof (for example a bearing) requiring lubrication. The lubricant fluid moves within a suitable conduit connecting a feed device for such fluid or said member.

A lubrication process which operates using the known MQL (minimum quantity lubrication) technology is also known and this comprises feeding a flow or spray phase or lubricating aerosol (comprising air and oil) controlled with regulated throughput and pressure (as regards both the air and the oil), which is delivered to said mechanical member (a tool for removing swarf) within a suitable conduit. Using this process a machine tool such as for example a drill can be lubricated in a precise manner, the lubricant reaching it in the form of spray drops.

Again in this case the sprayed lubricant fluid containing oil moves in a controlled way within the abovementioned conduit connecting the feed device for such fluid to the lubricated member. This conduit, especially in the case of a drill, is connected to a channel within the drill which opens at at least one hole provided on the surface of the drill to deliver the lubricant fluid to the machining element.

The abovementioned control, and in particular control of the throughput of lubricant (oil) present in the lubricating fluid, is achieved using various methods and devices, mainly of the optical type. For example a device for controlling throughput comprising an optical source (typically one or more LED) and a photodiode is known. The pipe or conduit transporting or feeding the sprayed lubricant flow (aerosol) is located between the source and the detector and the flow of oil present in that conduit can be derived using the light signal received by the photodiode.

In particular the systems implementing this technology make it possible to lubricate mechanical components and elements affected by friction with oil mist or minimum lubrication, such as for example the high-speed chuck ball bearings of machine tools used in machine shops, mills, reamers, grinders, drills, lathes and all bearings surrounding and supporting high speed rotating shafts, as well as machine tools which remove swarf.

The lubricant is present in the lubricant flow in the form of microdroplets of oil of the size of tenths of microns (a condition similar to cigarette smoke) within a carrier fluid (air) in pressurised conduits.

In this text by "lubricant flow" is meant the flow comprising air and lubricant (sprayed), or oil mist, while the term lubricant means the pure lubricant oil present in said lubricant fluid.

Precise control of the throughput of lubricant present in a lubricating conduit, whether in the liquid state or in the spray state, is an operation which is difficult to carry out using the solutions in the state of the art. This is because of the physical and hydraulic variables affecting the system and in particular the size of the lubricant particles if they are sprayed, the pressure of the working fluid, the conduit diameter, the velocity of the sprayed fluid, the colour of the flow, its viscosity and the like.

It is accepted that changes in the abovementioned hydraulic parameters are normal in MQL or oil mist lubrication systems for machine tools; for example these occur when the type of tool, which may have different dimensions and lubrication needs, is changed during machining.

This makes it necessary to have a very complex analysis of the lubricant measuring signal obtained by measuring the light diffused from the lubricant droplets (as acquired for example by a photodiode).

Hitherto the optical control of a stream of lubricant or oil aerosol/mist in the liquid state (streaking) moving within a transparent pipe has not actually provided a precise measurement of the flow of the metered lubricant, but only a check on the operation of the lubricant flow or fluid feed system or that oil is or is not present within the carrier fluid (air).

For example a minimum lubrication method and device according to which an optical sensor is used to detect the metered quantity of lubricant in an air-oil mixture (lubricant flow or fluid) within a pipe, a mixture which has to be fed to a lubricating member, are known from WO2010/128380.

WO2004079254 however describes a device for monitoring a lubricant flow or fluid, but one which is not appropriate for measuring throughput. This device, which always operates using means capable of detecting a change in the light emitted by a radiating element (for example a LED) and detected by a photodiode (in a direct manner in the case of this prior document) only provides a generic indication of the passage of fluid in a conduit, but is unable to provide an accurate determination of the throughput of the lubricant present therein, whether the latter is in the liquid state or in the spray state. The known solution therefore only makes it possible to detect whether or not lubricant is present in the fluid, but not the quantity (or throughput) of lubricant present in the fluid.

DE102006030651 describes a method and device for feeding a lubricant flow in spray form (air mist/aerosol) in a feed channel for working lubrication of a tool using the minimum quantity lubrication method.

In the known device the sprayed lubricant is monitored and consequently controlled through information derived from a flowmeter ("nebula flowmeter") capable of measuring the throughput of oil in the carrier fluid (air), a throughput which can be identified by the change in the intensity of a light beam (intercepted by said flow, as described above), caused by the particles of lubricant fluid passing across the detection target. Again this prior document does not however allow precise measurement of the lubricant throughput, but only provides a generic indication of whether it is or not present in the carrier fluid.

JP-A-201-2959889 and JP-A1302561 also describe detection of the throughput of fluid (quantity of sprayed fluid), which is not however measured directly, accurately and instantaneously as in DE102006030651. These Japanese texts also substantially only detect whether lubricant is or is not present in the carrier fluid, but do not allow a precise measurement of throughput.

US 2012/0004865 describes a method for measuring the average velocity of a blood fluid or a fluid for extracorporeal infusion using counter beam injection interferometry. The method comprises the emission of a first beam of laser light from the cavity of a semiconductor laser source, the reflection of a second laser beam from the fluid and the consequent generation of interference with the first laser beam within the laser cavity. The interference signal is detected using a detection photodiode and the interference signal detected is processed using an electronic processing and control circuit. An apparatus for implementing the aforesaid method and an extracorporeal circuit comprising said apparatus is also described.

According to this prior document a frequency spectrum for the signal emitted from the photodiode is determined and this spectrum is processed to determine a particular frequency that is proportional to the velocity of the monitored fluid. However the prior document takes into consideration a physical effect which is completely different from that relating to the measurement of a characteristic of a fluid (air) containing a lubricant. The prior document in fact measures the frequency shift due to the Doppler effect of a signal measured using a counter beam injection interferometer generated by a photodiode receiving light reflected from a single-phase fluid circulating in a conduit towards which light is generated from a laser. This known technique only makes it possible to measure the velocity of the fluid and is applied to a continuous flow of such fluid; this requires a laser source and a reflection measurement. This known solution does not measure the percentage of oil in a flow of compressed air (defining an oil mist) directed towards a lubricating member by measuring attenuation of the light transmitted through that two-phase fluid (air and oil).

In addition to this, the system or apparatus described in the prior document is complex and difficult to adjust mechanically when it is desired to attempt to overcome the disadvantages deriving from electronic processing of the signal emitted by the photodiode. Finally the prior document does not consider apparatus for spray lubrication.

Thus optical sensors for controlling lubricating systems (such as for example that described in DE 102006030651) which allow only generic control of the throughput of the oil or mixture containing it, which do not offer a precise measurement of throughput, but only make it possible to determine generically whether lubricant is present in the carrier liquid, are known.

These known systems also show limitations in their applications, such as for example the minimum metered value of lubricant that can be monitored and also the maximum diameter of the pipes monitored.

In addition, a further problem with known solutions is the need to use a specific detector device ("nebula flowmeter") which has to be inserted into the existing conduit for feeding the lubricant fluid. This results in such pipe being interrupted and cut, with obvious disadvantages (for example as regards leaks). Finally in known solutions the detector device has to be connected to a control unit external to the lubrication system.

The object of this invention is thus to provide a method and system for spray lubrication (of the mist or MQL type) which are improved, overcome the disadvantages of known control technologies and systems and in particular make possible the instantaneous control and precise measurement of the throughput of lubricant and/or its velocity, or at least one of the characteristics, throughput and velocity, of the lubricant.

Another object of the invention is to provide a system and method for accurate measurement of a property of a lubricant under spray lubrication conditions with a suitable variable feed pressure, changes in which result in a proportional change in the throughput of lubricant in a controlled or accurately measured way.

Another object of this invention is to provide a system and method for spray lubrication through mist or minimum lubrication allowing a stable continuous feed of lubricant independently of the variable back-pressure in the feed conduit for the air-oil mixture at the point which has to be lubricated.

Another object of this invention is that of providing a system and method for spray lubrication with an adjustable feed of the phases (air-oil) in terms of both air back pressure and volume of lubricant, and in which the lubricant feed is adjusted on the basis of measurement of the throughput of lubricant.

Another object of this invention is to provide a system and method for spray lubrication by means of mist or minimum lubrication using a sprayed air-oil lubricant flow that is capable of automatically indicating any deficit of lubricant pressure in the air-oil mixture which might cause functional problems with jamming of the bearing being lubricated.

These and other objects which will be apparent to those skilled in the art are accomplished through the method and system for minimum lubrication of mechanical members according to the appended claims.

For a better understanding of this invention there are attached, purely by way of example but without limitation, the following drawings in which:

> Figure 1 diagrammatically illustrates one embodiment of a mist lubrication system for bearings of a chuck according to the invention;
> Figure 2 diagrammatically illustrates one embodiment of an MQL lubricating system for a machine tool;
> Figure 3 shows a block diagram of a plan of the system in Figure 1;
> Figure 4 shows a graph of a plurality of spectra relating to different measurements of the composition of lubricant fluids containing different percentages of lubricant;
> Figure 5 shows a graph of a plurality of spectra or signal curves relating to different measurements of the composition of lubricant fluid containing a particular quantity of lubricant, but at different flow rates;
> Figures 6 and 7 show two graphs of spectra or signal curves obtained by implementing the present invention in two different cases of lubricant throughput in a carrier fluid;
> Figure 8 shows a graph of a spectrum obtained according to a variant of the invention.

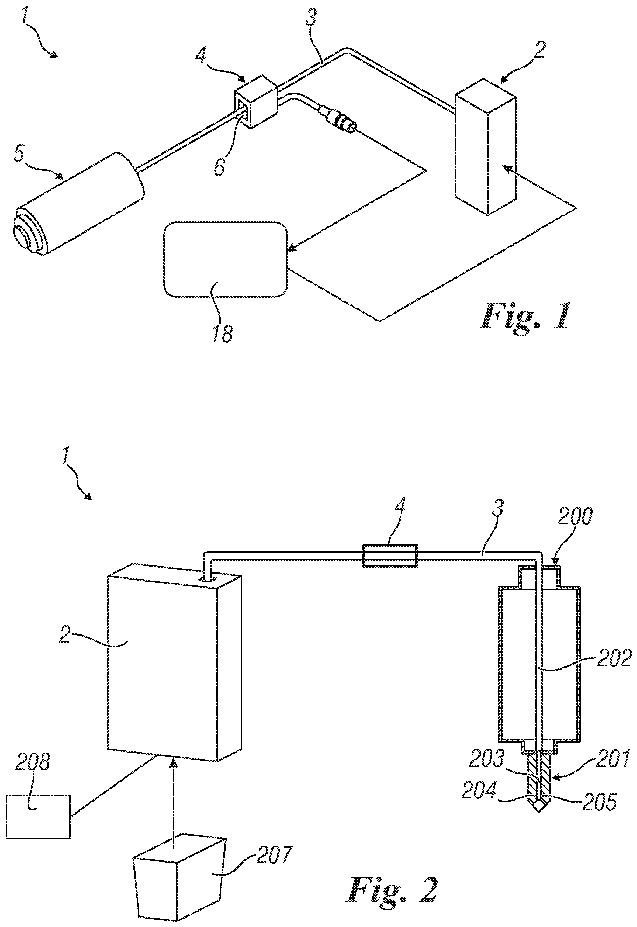

With reference to the aforementioned figures, Figure 1 illustrates by way of example a spray lubrication system 1 using mist lubrication according to the invention. In the example the lubricant and a carrier fluid (compressed air) are introduced from a dispensing spray device 2 into a conduit or pipe 3 which passes through a measurement device 4 before reaching a lubricating member 5 (for example a chuck of a machine tool).

Measurement device 4 has a seat 6 for such pipe which therefore passes through device 4 without suffering any interruption.

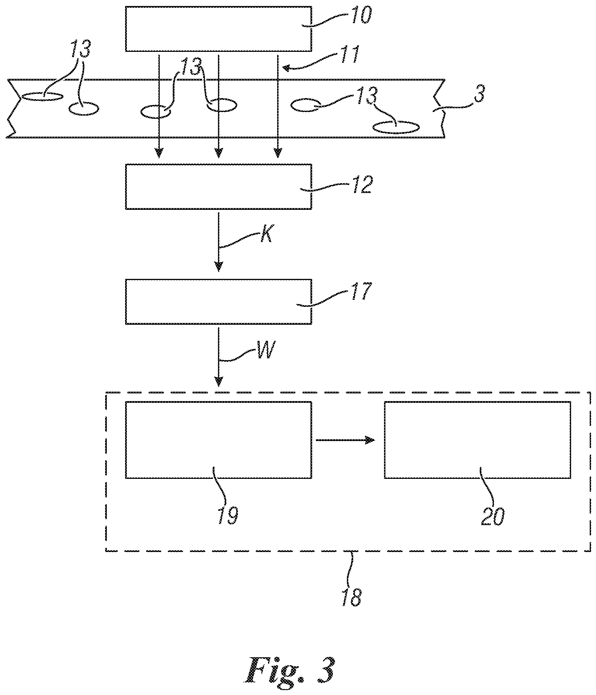

Measurement device 4 comprises (see Figure 3) an optical sensor having a generator 10 for a light beam 11, for example comprising one or more LEDs generating light in the infrared, and a detector 12 for such beam such as a photodiode or the like. Between these lies conduit 3 in which the droplets of lubricant (forming the mist) or oil 13 immersed in the carrier fluid move. Light beam 11 passes through conduit 3 (made of material which is transparent to infrared) projecting shadows onto detector 12 when it encounters oil droplets 13. The photodiode thus generates a signal that is proportional to the quantity of droplets (forming the mist), of a duration which depends on their velocity.

Figure 2 on the other hand illustrates a system 1 of MQL lubrication for machine tools using spray lubrication. In this figure, in which parts corresponding to those in Figure 1 are indicated by the same reference numbers, dispensing spray device 2 delivers spray lubricant for MQL lubrication to a tool holder member 200, such as a chuck, and from this enters tool 201 of the latter, for example a drill. This takes place through conduits 202 and 203 which are provided in the same member and in said tool. Conduit 203 opens onto the casing of the drill via two holes 204 and 205, as usual.

Device 2 is connected to a tank 207 and a control unit 208 for the operating machine, for example a digital control unit for the latter, which forms part of machine tool 200.

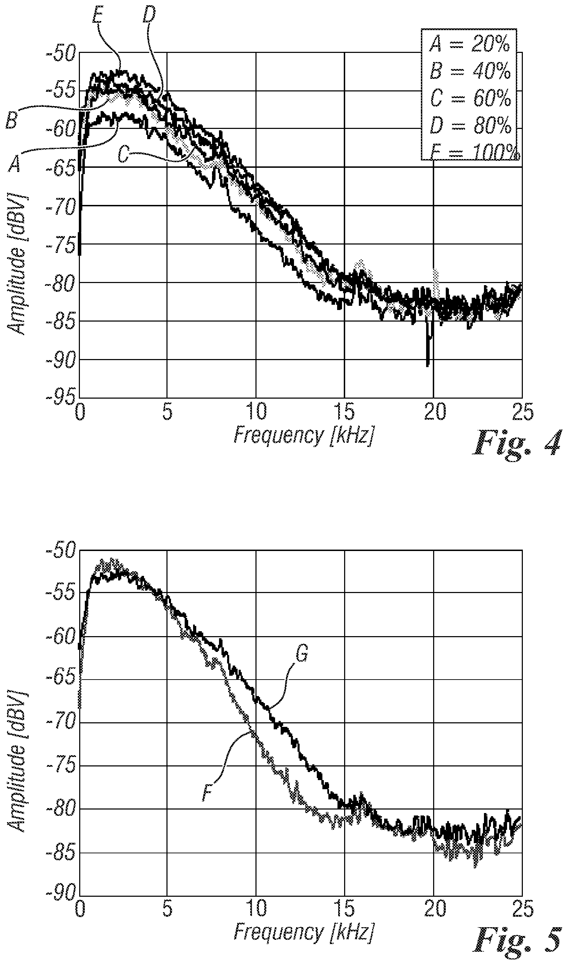

Conduit or pipe 3 connecting device 2 to machine tool 200 passes through measuring device 4 constructed as described above and having the functions mentioned above. The collection of numerous droplets produces a complex signal which can be represented by means of frequency/amplitude graphs such as those illustrated in Figures 4 and 5. This signal is provided by the sum of a very large number of contributions, each generated by each individual droplet of lubricant intercepted by light beam 11 before reaching detector 12.

The detector signal is therefore a "combination" of individual signals, which in the control techniques for spray lubrication in the state of the art appears as noise (noise-like signal).

In other words, as microdroplets of lubricant in suspension in the carrier fluid (compressed air) pass through conduit 3 within measuring device 4 the signal measured by detector or photodiode 12 provides a spectrum which varies according to the quantity of lubricant and the velocity of the lubricant fluid (defined by air and oil) . In accordance with the invention, using the signal spectrum measured, it is therefore possible to obtain a signal that is proportional to the precise quantity of lubricant carried by the conduit and its velocity, as will be described below.

Figure 4 illustrates an example of spectra acquired as the percentage of lubricant is varied, while Figure 5 shows an example of spectra acquired for the same amount of lubricant but at different flow rates. The complex signal produced by the photodiode will therefore comprise superimposed pulses (as indicated above) having amplitude and temporal duration characteristics that depend on the velocity of the drops and their number.

In the example, in Figure 4 signal curves A, B, C, D and E represent determinations made with a lubricant fluid (air + oil) containing 20%, 40%, 60%, 80% and 100% of lubricant respectively.

This percentage is calculated in respect of a maximum reference value of lubricant which can be present in the lubricant fluid in the spray phase (or in conduit 3 and carried by the air).

In Figure 5 on the other hand the curves have been obtained using the same quantity of lubricant or oil (for example 100% of the total lubricant fluid), but here the overall velocity of the lubricant fluid differs because it has been subjected to different pressures (curve F was obtained with a lubricant fluid subjected to a pressure of below 4 bar, while curve G was obtained with a lubricant fluid subjected to a pressure of over 4 bar).

Referring to Figure 3, photodiode 12 is connected to a current/voltage converter 17 which converts the current signal emitted by the photodiode (arrow K in Figure 3) into a voltage signal (arrow W in Figure 3) and delivers it to a control unit 18 which is advantageously and preferably a microprocessor connected directly to the photodiode, and possibly incorporated therewith. Through a processing unit 19 (which for example works by applying a Fourier transform to the signal originating from converter 17, which defines the power spectrum of the signal) and a further measurement device 20 operating according to specific algorithms (which will be described below) control unit 18 makes a precise measurement of at least one of the characteristics, flow and velocity, of the lubricant immersed in the lubricant fluid.

Through simulation of the system to which the invention relates, confirmed by experimental measurements, it has been found that the amplitude of the signal spectrum generated by photodiode 12 increases with the quantity of lubricant or oil 13 present in the lubricant fluid. The simulation considered a finite number of drops 13 passing across photodiode 12 per unit time. Each drop 13 gives rise to a signal pulse of duration which depends on the flow velocity and intensity which is dependent upon its cross-section.

It has been found that different velocities of the lubricant or oil 13 alters the shape of the spectrum or its duration, while a different quantity or a different flow rate changes only the amplitude of the signal.

Thus in order to control correct lubrication of member 5 through a precise quantity or throughput of lubricant, in accordance with the invention a frequency spectrum of the electrical signal emitted by photodiode 12 is determined and its amplitude (linked to the throughput of lubricant) and duration (linked to the velocity of the lubricant in the lubricating fluid) are evaluated; it is thus possible to make an instantaneous determination of the lubrication condition of member 5, and if necessary change it. In this case in fact control unit 18 which is connected to the device dispensing lubricant fluid may act on the latter to change its operating conditions (for example by increasing the pressure of the air in the aforesaid fluid or changing the percentage of oil present in said fluid) and optimise lubrication of member 5.

It is therefore important that the signal emitted by the photodiode (which is complex because it derives from a combination of the individual signals generated by each droplet of lubricant or oil present in the carrier fluid) should be effectively defined or measured.

As mentioned, this signal is first processed by processing unit 19; in particular the complex signal (which with the known techniques in the state of the art is detected as generic noise due to the presence of a quantity of lubricant that is not well defined) is broken down into its components, preferably and advantageously through the use of a Fourier transform. This then makes it possible to identify the lubrication parameters in a very accurate way.

Through the Fourier transform the signal detected or its waveform (which is usually very complex and "noisy") can be broken down into several components, so that its true composition can be understood. More specifically the Fourier transform makes it possible to calculate the different components (amplitude, phase and frequency) of sinusoidal waves, which when summed together give rise to the original signal generated by photodiode 12.

By performing the Fourier transform the amplitude and frequency values of all the waves making it up are obtained, and thus it is possible to analyse the "internal" details of the signal in question and recognise and precisely measure the quantity or throughput of oil in the lubricant fluid. In addition to this, by observing the various components of the signal it is possible to "filter" the wave (representing the aforesaid signal), removing those frequencies considered to be disturbance.

Once the signal has been transformed as indicated above measuring device 20 subjects the signal to an analysis using different measurement algorithms.

These algorithms will be indicated below.

Measurement of lubricant flow under constant hydraulic conditions or at constant velocity of the lubricating fluid.

1. A value proportional to the precise quantity or throughput of lubricant per unit time can be obtained from an estimate of the power of the electrical signal generated by photodiode 12 and processed by processing unit 19. The power is obtained by making a linear summation of the values in the power spectrum. In order to obtain a more accurate zero value it is initially necessary to subtract the value obtained without any flow of lubricant fluid in conduit 3 while using the same optical-mechanical system. Also it is preferable to limit the summation to the maximum frequency of the signal (typically around tens of kHz for typical microlubrication circuits), that is so that the signal values are higher than the background noise. Control unit 18 constructed in this way requires only one calibration measurement in order to obtain the proportionality coefficient between the value of the actual flow of lubricant fluid and the result of the summation of the spectrum values.

This algorithm provides correct values if the hydraulic circuit is not changed (same pressures and same output mechanics) and can be effectively applied even to low signal-to-noise ratios.

2. Measurement of flow and velocity

Through a more complex analysis of the spectrum it is possible to determine the precise quantity or throughput of oil present in the conduit (proportional to the height of the "spectrum bell" or the amplitude of the signal) and the flow velocity (associated with the width of the "spectrum bell" or the signal duration).

These analyses may be performed using different techniques, some examples of which will be described below.

2.1 Regression curves

A first processing technique comprises finding the minimum squares regression curve for two parameters: amplitude and frequency width fo. This is done using the following formula:

$$regression\ line = \frac{A}{\left(1 + if / f_0\right)^6}$$

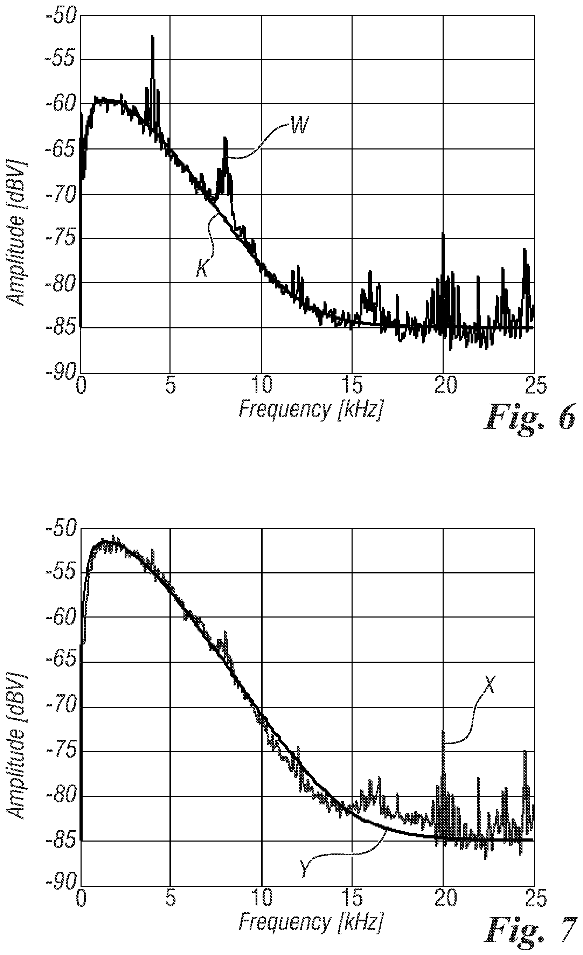

The signal or curve obtained is filtered using a highpass filter and then added to the background noise.
One example of the application of this method is illustrated in Figure 6, which was obtained by analysing the signal generated by a lubricant fluid subjected to regression analysis with the following A and F values: A=0.0014, $f_0$=9 kHz.
Curve W represents the detected signal and curve K represents the processed signal. This signal proved to be optimally consistent with an oil throughput of 20% of lubricant fluid.
A second example is shown in Figure 7 where the signal detected was processed with A=0.032 and $f_0$=9 kHz. Two curves are again shown in this figure: curve X represents the signal acquired from the photodiode (and reprocessed by processing unit 19), and curve Y shows the signal processed by measuring device 20. What was obtained was proved to be optimally consistent with oil present at 100% in the lubricant fluid. It should be noted that curves K and Y have the same frequency (that is deriving from the same lubricant fluid pressure), while the square of the ratio between the amplitudes (0.032/0.014) amounts to 5 (ratio between 100% and 20%). This relationship is found for all the measurements made.
Amplitude is a function of the percentage of oil, while width is a function of the flow velocity. With suitable calibration of member 20 it is found that $A^2$ becomes proportional to the quantity of oil, while $f_0$ is proportional to the velocity; $A^2 * f_0$ is therefore proportional to the overall throughput of oil in the lubricant fluid.
This technique is very powerful but it requires a large calculation capacity for processing in real time.

2.2 Peak evaluation

Simpler regression techniques can be applied to estimate the maximum value of the frequency spectrum, which will be proportional to the percentage of oil present in the sprayed fluid. The simplest technique comprises direct measurement of the maximum value, a technique which is not very accurate, but is quick. To improve performance the maximum may be measured after operations for obtaining the mean of the spectrum and filtering of the curve (for example with a moving mean). As an alternative a mean may be obtained from a particular number of low frequency samples. This can be done for example using the following formula:

$$Vpeak = \frac{1}{n} \sum_{i=1}^{n} S(f_i)$$

where n is intended for example to be up to 5 kHz, with reference to Figure 2.

2.3. Barycentre of the curve

A third processing technique consists of calculating the barycentre of the spectrum, obtained using a linear scale or a logarithmic scale after having subtracted the background noise. The value of the barycentre for frequency will be a regular function of the flow velocity, while the peak of the curve will again be a function of the percentage of oil.

2.4 Evaluation of bandwidth

A fourth processing technique comprises evaluating the bandwidth of the frequency signal, for example at -3 dB. The frequency corresponding to a fall of 3 dB with respect to the summit of the spectrum will be a function of the flow velocity, while the apex of the curve will be a function of the quantity of oil.
It will therefore be seen from the above that the invention makes it possible to obtain an evaluation of the actual quantity or actual flow of oil in the lubricant fluid and a velocity of the lubricant, in real time. This is achieved through an analysis of the spectrum of the signal emitted by optical detector 12 of measuring device 15. This analysis is able to take into consideration and analyse the power of the signal using its power spectrum; the amplitude and width of the frequency spectrum from the signal emitted by optical detector 12; the maximum of the spectral curve; the apex of the spectral curve and its frequency.
The solution according to the invention may appear to be similar to that in US2012/0004865. However the prior document considers a physical effect which is completely different from that described above and proposed by the present invention. The prior document only allows for measurement of velocity and is applied to a continuous flow of fluid, requiring a laser source and a reflection measurement. On the contrary the present invention measures the percentage of oil in a flow of compressed air (said mist) through measuring the attenuation of the transmitted light. Thus it does not require that the light source should have coherence properties (a laser is not required) and it is therefore capable of measuring both the concentration of oil in air (or the throughput) and its velocity. Analogy with the prior document relates to the shape of the spectrum of the signal at the photodetector in response to a flow of fluid, which may appear to be similar. However, given that the physical phenomenon is completely different, its information content is different and as such will be processed using different techniques, even though based on spectral analysis.

According to one variant of the invention it is possible to determine the throughput of lubricant and its velocity through measuring the continuous component of the signal detected by photodiode 4 by normalising the spectrum values. By dividing the spectral values as determined in the previous figures by the value of the signal itself continuously the measurement system becomes independent of fluctuations in the power of source or generator 10 (for example due to aging or replacement of the component), the sensitivity of detector 12 (associated for example with changes in temperature or mechanical alignment) and reduction (due for example to changes in the transparency of the conduit, any marks on the conduit or dirt).

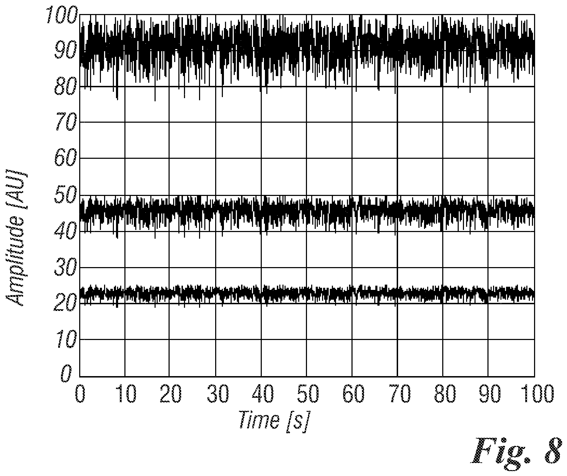

Figure 8 illustrates this concept graphically: the signal due to the passage of oil in the form of a mist increases in amplitude proportionally to the continuous signal (which would be present even in the absence of a flow of oil). Normalisation of the spectrum with respect to the continuous component of the signal renders the system more robust to changes in amplitude of the signal and makes it possible to detect characteristics of the oil (throughput and velocity) which are independent of external factors associated with the mechanical, optical and measurement problems mentioned above.

In Figure 8 the measurement curves over time have been obtained by reducing the power of generator 10 from 100% to 50% and 25%. It will be seen that the modulation signal due to the passage of oil varies proportionately to the continuous value.

The method and system according to the invention may also be used to diagnose (in the sense of monitoring) whether the MQL or mist lubrication devices are functioning properly. In fact control unit (or microprocessor) 18 continuously evaluates the signal originating from detector or photodiode 12 and monitors it over time. Whenever such unit 18 finds that the signal amplitude falls over time (percentage with respect to an initially detected value, a predetermined value or one below a signal value), this unit determines that the throughput (or quantity) of lubricant in the lubricating fluid is falling. This may for example be due to malfunctioning of device 2 or emptying of oil tank 207.

At the same time, if unit 18 finds that the signal originating from photodiode 12 changes its frequency range (or width) over time, this unit can detect a fall in the velocity of the lubricant and therefore a fall in the pressure of the lubricant fluid as a whole, or of the air present in the latter.

Thus thanks to control of the signal emitted by the photodiode it is possible to perform a diagnosis on the system with regard to both oil feed and air pressure.

Various embodiments of the invention have been described and referred to. Yet others are however possible: for example the signal generated by photodiode 12 may be analysed using not only the means described, but also using equivalent means known to those skilled in the art. The invention should not therefore be regarded as being limited to the aforesaid means described, but as defined by the appended claims.

## Claims

1. Method for measuring flow rate and velocity of a lubricant in an oil mist moving within a conduit (3), said oil mist comprising compressed air and said lubricant, said oil mist being used in a process for lubricating components, mechanical members (5) or tools for machining operations, said method comprising detecting the flow rate and pressure of such lubricant, such flow rate determination being performed using optical sensor means (4) comprising a generator (10) of a light beam (11) and a detector (12) of such beam (11), the oil mist moving between said generator (10) and said detector (12), the presence of lubricant altering the light beam (11) striking said detector (12) in such a way as to modify an electrical signal generated by such detector (12), said measurement being made in real time, a provision being made for monitoring the frequency range of a frequency spectrum of said electrical signal emitted by said detector (12) in order to detect the velocity of the lubricant in the oil mist, **characterised in that** the amplitude of said frequency spectrum is monitored in order to measure the flow rate of the lubricant in the oil mist from the analysis of said amplitude, the electrical signal generated by the detector (12) being broken down into a combination of individual signals having amplitude and temporal duration characteristics which are different from each other and which vary as a function of the quantity of lubricant present in the oil mist and as a function of the velocity of such oil mist, the amplitude and frequency values of each individual signal being calculated, said amplitude and frequency values so obtained being correlated with each one of the flow rate and velocity of the oil present in such fluid, the analysis of the signal being performed under constant hydraulic conditions.

2. Method according to claim 1, **characterised in that** the light beam is generated by a LED located on one side of the conduit (3), the optical sensor means being located on the other side of said conduit.

3. Method according to claim 1, **characterised in that** provision is made for processing the signal leaving the detector (12) of the light beam (11) converting it into a voltage, and subsequently for obtaining a spectrum of the converted signal through a mathematical transform, said spectrum being thus broken up into the individual signals of which it is made up.

4. Method according to claim 3, **characterised in that** the electrical signal is processed using a Fourier transform.

5. Method according to claim 1, **characterised in that** provision is made for linear summation of the power spectrum of the electrical signal, said summation being limited to the maximum frequency of the signal or so that the signal values are higher than the background noise.

6. Method according to claim 3, **characterised in that** at least one of the amplitude and frequency characteristics of the signal is evaluated using a minimum squares regression curve or alternatively by means of evaluating the summit value of the frequency spectrum.

7. Method according to claim 3, **characterised in that** at least one of the amplitude and frequency characteristics of the signal is evaluated by calculating the barycentre of the frequency spectrum.

8. Method according to claim 3, **characterised in that** when applying the bandwidth method a threshold value of the signal is selected and the corresponding value of the frequency of such signal with respect to the summit is evaluated so as to determine the velocity of the oil flow.

9. Method according to claim 1, **characterised in that** provision is made for using measurement of the characteristic of the frequency spectrum of the electrical signal generated by the detector (12) of the light beam (11) to perform a diagnosis of the feed of oil mist and in particular the presence of a correct quantity or flow rate of oil **in that** oil mist and a correct pressure of such oil mist.

10. System for measuring the flow rate and velocity of a lubricant in an oil mist moving within a conduit (3), said oil mist comprising compressed air and said lubricant, said oil mist being used in a process for the lubrication of components, mechanical members (5) or tools for mechanical machining, the system implementing the method according to claim 1, said system comprising means (4, 18) for monitoring and adjusting the flow rate and pressure of such lubricant in the oil mist, these control means comprising optical sensor means (4) having a generator (10) of a light beam (11) and a detector (12) for such beam (11), the oil mist passing between said generator (10) and said detector (12), the presence of lubricant modifying the light beam (11) striking said detector (12), provision being made for monitoring means (18) directly connected to the optical sensor means (4) capable of determining the aforesaid characteristic of the lubricant in real time, said monitoring means (18) receiving an electrical signal from said optical sensor means (4) which is a function of the measurement made of the presence of lubricant (3) in the lubricant fluid, said monitoring means (18) generating a frequency spectrum of such signal and determining the characteristic of the lubricant from an analysis of such spectrum, provision being made for a unit (19) processing the electrical signal emitted by the optical sensor means (4), a frequency range of a frequency spectrum of said signal being monitored in order to detect the velocity of the lubricant in the oil mist, **characterised in that** the amplitude of said frequency spectrum is detected in order to measure the flow rate of the lubricant in the oil mist from the analysis of said amplitude, said unit breaking down said electrical signal into a plurality of individual electrical signals making it up, the amplitude and frequency values of each of such individual electrical signals which are correlated with the flow rate and velocity of the lubricant in the oil mist being then calculated.

11. System according to claim 10, **characterised in that** said monitoring means comprise a measuring device (20) for the amplitude of the frequency spectrum of the electrical signal generated by said optical sensor means (4).

12. System according to claim 10, **characterised in that** said monitoring means are a microprocessor unit.

13. System according to claim 10, **characterised in that** said generator of a light beam being a LED (10) located on one side of the conduit (3), the optical sensor means (4) being located on the other side of the conduit.

## Patentansprüche

1. Verfahren zum Messen Durchflussmenge und -geschwindigkeit eines Schmiermittels in einem Ölnebel, der sich in einer Leitung (3) bewegt, wobei der Ölnebel Druckluft und das Schmiermittel umfasst, wobei der Ölnebel in einem Prozess zum Schmieren von Komponenten, mechanischen Elementen (5) oder Werkzeugen für Vorgänge der maschinellen Bearbeitung verwendet wird, wobei das Verfahren das Erkennen der Durchflussmenge und des Drucks des Schmiermittels umfasst, wobei die Bestimmung der Durchflussmenge mit Hilfe optischer Sensormittel (4) ausgeführt wird, die einen Generator (10) eines Lichtstrahls (11) und einen Detektor (12) dieses Strahls (11) umfassen, wobei sich der Ölnebel zwischen dem Generator (10) und dem Detektor (12) bewegt, wobei das Vorhandensein von Schmiermittel den Lichtstrahl (11), der auf den Detektor (12) trifft, derart verändert, dass ein elektrisches Signal modifiziert wird, das durch den Detektor (12) erzeugt wird, wobei die Messung in Echtzeit vorgenommen wird, wobei vorgesehen ist, den Frequenzbereich eines Frequenzspektrums des von dem Detektor (12) ausgestrahlten elektrischen Signals zu überwachen, um die Geschwindigkeit des Schmiermittels in dem Ölnebel zu erkennen, **dadurch gekennzeichnet, dass** die Amplitude des Frequenzspektrums überwacht wird, um die Durchflussmenge des Schmiermittels in dem Ölnebel durch die Analyse der Amplitude zu messen, wobei das von dem Detektor (12) erzeugte elektrische Signal in eine Kombination aus einzelnen Signalen unterteilt wird, die Amplituden- und Zeitdauereigenschaften aufweisen, die sich voneinander unterscheiden und die als eine Funktion der Menge des Schmiermittels, das in dem Ölnebel vorhanden ist, und als eine Funktion der Geschwindigkeit dieses Ölnebels variieren, wobei die Amplituden- und Frequenzwerte jedes einzelnen Signals berechnet werden, wobei die so erzielten Amplituden-und Frequenzwerte mit jeweils der Durchflussmenge und der Geschwindigkeit des in diesem Fluid vorhandenen Öls korrelieren, wobei die Analyse des Signals unter konstanten hydraulischen Bedingungen ausgeführt wird.

2.  Verfahren nach Anspruch 1, **dadurch gekennzeichnet, dass** der Lichtstrahl durch eine LED erzeugt wird, die sich an einer Seite der Leitung (3) befindet, wobei sich die optischen Sensormittel an der anderen Seite der Leitung befinden.

3.  Verfahren nach Anspruch 1, **dadurch gekennzeichnet, dass** vorgesehen ist, das Signal zu verarbeiten, das den Detektor (12) des Lichtstrahls (11) verlässt, selbiges in eine Spannung umzuwandeln und danach ein Spektrum des umgewandelten Signals durch eine mathematische Transformation zu erzielen, wobei das Spektrum somit in die individuellen Signale getrennt wird, aus denen es besteht.

4.  Verfahren nach Anspruch 3, **dadurch gekennzeichnet, dass** das elektrische Signal mit Hilfe einer Fourier-Transformation verarbeitet wird.

5.  Verfahren nach Anspruch 1, **dadurch gekennzeichnet, dass** eine lineare Summation des Leistungsspektrums des elektrischen Signals vorgesehen ist, wobei die Summation auf die maximale Frequenz des Signals begrenzt ist oder derart, dass die Signalwerte größer als das Hintergrundrauschen sind.

6.  Verfahren nach Anspruch 3, **dadurch gekenn-** zeichnet, dass mindestens eine der Amplituden- und Frequenzeigenschaften des Signals mit Hilfe einer Kleinste-Quadrate-Regressionskurve oder alternativ mittels Bewerten des Spitzenwertes des Frequenzspektrums bewertet wird.

7.  Verfahren nach Anspruch 3, **dadurch gekennzeichnet, dass** mindestens eine der Amplituden- und Frequenzeigenschaften des Signals durch Berechnen des Baryzentrums des Frequenzspektrums bewertet wird.

8.  Verfahren nach Anspruch 3, **dadurch gekennzeichnet, dass**, wenn das Bandbreitenverfahren angewandt wird, ein Grenzwert des Signals ausgewählt wird und der entsprechende Wert der Frequenz dieses Signals im Verhältnis zum Spitzenwert bewertet wird, um die Geschwindigkeit des Ölstroms zu bestimmen.

9.  Verfahren nach Anspruch 1, **dadurch gekennzeichnet, dass** vorgesehen ist, die Messung der Eigenschaft des Frequenzspektrums des elektrischen Signals, das durch den Detektor (12) des Lichtstrahls (11) erzeugt wird, zu verwenden, um eine Diagnose des Einspeisens von Ölnebel und insbesondere des Vorhandenseins einer korrekten Menge oder Durchflussmenge von Öl in dem Ölnebel und eines korrekten Drucks dieses Ölnebels auszuführen.

10.  System zum Messen der Durchflussmenge und -geschwindigkeit eines Schmiermittels in einem Ölnebel, der sich in einer Leitung (3) bewegt, wobei der Ölnebel Druckluft und das Schmiermittel umfasst, wobei der Ölnebel in einem Prozess zum Schmieren von Komponenten, mechanischen Elementen (5) oder Werkzeugen für die maschinelle Bearbeitung verwendet wird, wobei das System das Verfahren nach Anspruch 1 umsetzt, wobei das System Mittel (4, 18) zum Überwachen und Justieren der Durchflussmenge und des Drucks des Schmiermittels in dem Ölnebel umfasst, wobei diese Steuermittel optische Sensormittel (4) mit einem Generator (10) eines Lichtstrahls (11) und einem Detektor (12) dieses Strahls (11) aufweisen, wobei der Ölnebel zwischen dem Generator (10) und dem Detektor (12) hindurchzieht, wobei das Vorhandensein von Schmiermittel den Lichtstrahl (11) modifiziert, der auf den Detektor (12) trifft, wobei Überwachungsmittel (18) vorgesehen sind, die direkt mit den optischen Sensormitteln (4) verbunden und in der Lage sind, die zuvor genannte Eigenschaft des Schmiermittels in Echtzeit zu bestimmen, wobei die Überwachungsmittel (18) ein elektrisches Signal von den optischen Sensormitteln (4) empfangen, das eine Funktion der erfolgten Messung des Vorhandenseins von Schmiermittel (3) in dem Schmiermittelfluid ist, wobei die Überwachungsmittel (18) ein Frequenzspektrum dieses Signals erzeugen und die Eigenschaft des Schmiermittels aus einer Analyse dieses Spektrums bestimmen, wobei eine Einheit (19) vorgesehen ist, die das von den optischen Sensormitteln (4) ausgestrahlte Signal verarbeitet, wobei ein Frequenzbereich eines Frequenzspektrums des Signals überwacht wird, um die Geschwindigkeit des Schmiermittels in dem Ölnebel zu erkennen, **dadurch gekennzeichnet, dass** die Amplitude des Frequenzspektrums erkannt wird, um die Durchflussmenge des Schmiermittels in dem Ölnebel durch die Analyse der Amplitude zu messen, wobei die Einheit das elektrische Signal in mehrere einzelne Signale unterteilt, aus denen es besteht, wobei dann die Amplituden- und Frequenzwerte jedes dieser einzelnen elektrischen Signale, die mit der Durchflussmenge und der Geschwindigkeit des Schmiermittels in dem Ölnebel korrelieren, berechnet werden.

11. System nach Anspruch 10, **dadurch gekennzeichnet, dass** die Überwachungsmittel ein Messgerät (20) für die Amplitude des Frequenzspektrums des von den optischen Sensormitteln (4) erzeugten elektrischen Signals umfassen.

12. System nach Anspruch 10, **dadurch gekennzeichnet, dass** die Überwachungsmittel eine Mikroprozessoreinheit sind.

13. System nach Anspruch 10, **dadurch gekennzeichnet, dass** der Generator des Lichtstrahls eine LED (10) ist, die sich an einer Seite der Leitung (3) befindet, wobei sich die optischen Sensormittel (4) an der anderen Seite der Leitung befinden.


**Revendications**

1. Procédé de mesure du débit et de la vitesse d'un lubrifiant dans un brouillard d'huile se déplaçant à l'intérieur d'un conduit (3), ledit brouillard d'huile comprenant de l'air comprimé et ledit lubrifiant, ledit brouillard d'huile étant utilisé dans un procédé de lubrification de composants, d'éléments mécaniques (5) ou d'outils pour des opérations d'usinage, ledit procédé comprenant la détection du débit et de la pression de ce lubrifiant, cette détection du débit étant effectuée à l'aide des moyens de détection optique (4) comprenant un générateur (10) d'un faisceau lumineux (11) et un détecteur (12) de ce faisceau (11), le brouillard d'huile se déplaçant entre ledit générateur (10) et ledit détecteur (12), la présence de lubrifiant modifiant le faisceau lumineux (11) frappant ledit détecteur (12) de manière à modifier un signal électrique généré par ce détecteur (12), ladite mesure étant effectuée en temps réel, une disposition étant prise pour surveiller la gamme de fréquences d'un spectre de fréquences dudit signal électrique émis par ledit détecteur (12) afin de détecter la vitesse du lubrifiant dans le brouillard d'huile, **caractérisé en ce que** l'amplitude dudit spectre de fréquence est surveillée afin de mesurer le débit du lubrifiant dans le brouillard d'huile à partir de l'analyse de ladite amplitude, le signal électrique généré par le détecteur (12) étant décomposé en une combinaison de signaux individuels ayant des caractéristiques d'amplitude et de durée temporelle différentes les unes des autres et qui varient en fonction de la quantité de lubrifiant présent dans le brouillard d'huile et en fonction de la vitesse de ce brouillard d'huile, les valeurs d'amplitude et de fréquence de chaque signal individuel étant calculées, lesdites valeurs d'amplitude et de fréquence ainsi obtenues étant corrélées avec chacun des débit et vitesse de l'huile présentes dans ce fluide, l'analyse du signal étant effectuée dans des conditions hydrauliques constantes.

2. Procédé selon la revendication 1, **caractérisé en ce que** le faisceau lumineux est généré par une LED située sur un côté du conduit (3), les moyens de détection optique étant situés sur l'autre côté dudit conduit.

3. Procédé selon la revendication 1, **caractérisé en ce qu'**il est prévu de traiter le signal sortant du détecteur (12) du faisceau lumineux (11) en le convertissant en une tension, puis d'obtenir un spectre du signal converti par une transformée mathématique, ledit spectre étant ainsi décomposé en des signaux individuels qui le composent.

4. Procédé selon la revendication 3, **caractérisé en ce que** le signal électrique est traité à l'aide la transformée de Fourier.

5. Procédé selon la revendication 1, **caractérisé en ce qu'**il est prévu une sommation linéaire du spectre de puissance du signal électrique, ladite sommation étant limitée à la fréquence maximale du signal ou de sorte que les valeurs du signal soient supérieures au bruit de fond.

6. Procédé selon la revendication 3, **caractérisé en ce qu'**au moins l'une des caractéristiques d'amplitude et de fréquence du signal est évaluée à l'aide d'une courbe de régression des carrés minimaux ou alternativement au moyen d'évaluation de la valeur maximale du spectre de fréquence.

7. Procédé selon la revendication 3, **caractérisé en ce qu'**au moins l'une des caractéristiques d'amplitude et de fréquence du signal est évaluée en calculant le barycentre du spectre de fréquence.

8. Procédé selon la revendication 3, **caractérisé en ce**

**que** lors de l'application de la méthode de largeur de bande, une valeur de seuil du signal est sélectionnée et la valeur correspondante de la fréquence de ce signal par rapport au sommet est évaluée de manière à déterminer la vitesse d'écoulement de l'huile.

9. Procédé selon la revendication 1, **caractérisé en ce qu'**il est prévu d'utiliser la mesure de la caractéristique du spectre de fréquence du signal électrique généré par le détecteur (12) du faisceau lumineux (11) pour effectuer un diagnostic de l'alimentation en brouillard d'huile et en particulier de la présence d'une quantité ou d'un débit d'huile correct dans ce brouillard d'huile et d'une pression correcte de ce brouillard d'huile.

10. Système de mesure du débit et de la vitesse d'un lubrifiant dans un brouillard d'huile se déplaçant à l'intérieur d'un conduit (3), ledit brouillard d'huile comprenant de l'air comprimé et ledit lubrifiant, ledit brouillard d'huile étant utilisé dans un procédé de lubrification de composants, d'éléments mécaniques (5) ou d'outils d'usinage mécanique, le système mettant en œuvre le procédé selon la revendication 1, ledit système comprenant des moyens (4, 18) de contrôle et de réglage du débit et de la pression de ce lubrifiant dans le brouillard d'huile, ces moyens de contrôle comprenant des moyens de détection optiques (4) ayant un générateur (10) d'un faisceau lumineux (11) et un détecteur (12) de ce faisceau (11), le brouillard d'huile passant entre ledit générateur (10) et ledit détecteur (12), la présence de lubrifiant modifiant le faisceau lumineux (11) frappant ledit détecteur (12), des moyens de contrôle (18) étant prévu directement connectés aux moyens de détection optiques (4) capables de déterminer en temps réel la caractéristique précitée du lubrifiant, lesdits moyens de contrôle (18) recevant un signal électrique desdits moyens de détection optiques (4) qui est une fonction de la mesure effectuée de la présence de lubrifiant (3) dans le fluide lubrifiant, lesdits moyens de contrôle (18) générant un spectre de fréquence de ce signal et déterminant la caractéristique du lubrifiant à partir d'une analyse de ce spectre, une unité (19) traitant le signal électrique émis par les moyens de détection optiques (4) étant prévue, une plage de fréquence d'un spectre de fréquence dudit signal étant surveillée afin de détecter la vitesse du lubrifiant dans le brouillard d'huile, **caractérisé en ce que** l'amplitude dudit spectre de fréquence est détectée afin de mesurer le débit du lubrifiant dans le brouillard d'huile à partir de l'analyse de ladite amplitude, ladite unité décomposant ledit signal électrique en une pluralité de signaux électriques individuels le composant, les valeurs d'amplitude et de fréquence de chacun de ces signaux électriques individuels qui sont corrélées avec le débit et la vitesse du lubrifiant dans le brouillard d'huile étant ensuite calculées.

11. Système selon la revendication 10, **caractérisé en ce que** lesdits moyens de surveillance comprennent un dispositif de mesure (20) de l'amplitude du spectre de fréquence du signal électrique généré par lesdits moyens de capteur optique (4).

12. Système selon la revendication 10, **caractérisé en ce que** lesdits moyens de surveillance sont un microprocesseur.

13. Système selon la revendication 10, **caractérisé en ce que** ledit générateur d'un faisceau lumineux est une LED (10) située sur un côté du conduit (3), les moyens de détection optiques (4) étant situés sur l'autre côté du conduit.

*Fig. 1*

*Fig. 2*

**Fig. 3**

A = 20%
B = 40%
C = 60%
D = 80%
E = 100%

*Fig. 4*

*Fig. 5*

*Fig. 6*

*Fig. 7*

Fig. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010128380 A
- WO 2004079254 A
- DE 102006030651
- JP 2012959889 A
- JP 302561 A
- US 20120004865 A